# FASCICULE DE BREVET EUROPEEN

(11) **EP 1 051 979 B1**
(45) Date de publication et mention de la délivrance du brevet: **04.11.2009**
(21) Numéro de dépôt: 00401257.1
(22) Date de dépôt: 09.05.2000
(51) Int. Cl.: A61K 47/44, A61K 31/60, A61K 31/505, A61K 31/455, A61K 31/20, A61Q 19/00

(54) **Utilisation de lipides peroxydés pour prévenir et/ou traiter les effets irritants d'un principe actif**
Verwendung von Peroxidlipiden zur Vorbeugung und/oder Behandlung von irritierenden Effekten eines Wirkstoffes
Use of peroxidised lipids to prevent and/or treat the irritating effect of an active agent

(30) Priorité: 12.05.1999 FR 9906079
(43) Date de publication de la demande: 15.11.2000
(73) Titulaire: LABORATOIRE CARILENE, 78360 Montesson (FR)
(72) Inventeur: Desjonqueres, Stéphane, 78600 Maisons-Laffitte (FR)
(74) Mandataire: Giraud, Françoise

(56) Documents cités:
- EP-A- 0 225 831
- EP-A- 0 391 780
- EP-A- 0 465 313
- EP-A- 0 481 148
- WO-A-97/26892
- FR-A- 2 750 331
- FR-A- 2 753 374

## Description

La présente invention concerne une nouvelle utilisation des lipides peroxydés comme agents destinés à prévenir et/ou à traiter les effets irritants d'un principe actif.

Il est bien connu qu'un certain nombre de principes actifs destinés à être utilisés par voie topique sur la peau, notamment de principes actifs utilisés en dermatologie, sont susceptibles de générer des problèmes sévères ou même très sévères de tolérance. Les effets secondaires de ces principes actifs peuvent aller de la simple irritation jusqu'à des manifestations de type eczémateuse, des risques de cloques ou de vésicules sur la peau.

Un exemple de produit bien connu provoquant de tels effets est la capsicine qui répond à la formule : 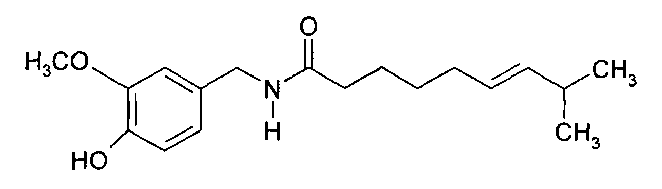 Il s'agit d'un produit bien connu pour son action vasodilatatrice. Grâce à cette action, la capsicine est largement utilisée dans des préparations à usage local dont l'effet est de procurer un afflux de sang et une sensation de chaleur. A ce titre, la capsicine est tout particulièrement indiquée dans le traitement des douleurs en rhumatologie. Un des inconvénients toutefois de ce produit est qu'il s'agit d'un produit irritant qui provoque des picotements sur la peau.

Un autre exemple de produit également connu pour son caractère particulièrement irritant est constitué de l'acide rétinoïque encore appelé vitamine A acide, principe actif particulièrement utilisé dans des compositions topiques destinées au traitement de l'acné et des rides.

Enfin d'autres principes actifs sont bien connus également pour leur caractère irritant. On citera en particulier les rétinoïdes de synthèse, le 5-fluoro uracyl, le peroxyde de benzoyle ainsi que la nicotinamide et l'acide salicylique.

On connaît différents lipides peroxydés, notamment obtenus par peroxydation d'huile végétale naturelle. On citera, en particulier, les brevets suivants BSM N°2 330 M, EP-A-293 535, FR-A-2 591 112, EP-A-225 831, EP-A-225 832, EP-A-225 833, EP-A-226 506, FR-A-2 461 744, FR-A-2 539 142 et EP-A-1-117 962 qui concernent soit la préparation de tels lipides peroxydes soit leurs applications dans différents domaines, en particulier dans le traitement de certaines affections dans le domaine de la rhumatologie ou de la traumatologie, ou encore en tant que produit cicatrisant.

La demande française FR 2 750 331 décrit des compositions cosmétiques à base de corps gras peroxydés comprenant un ou plusieurs corps gras peroxydés et contenant en outre, un ou plusieurs corps gras non peroxydés en quantité comprise entre 10 et 50%. Ce document concerne également l'application des compositions qui y sont décrites pour la prévention des escarres, des troubles de la micro-circulation et des états pelliculaires. Dans ces compositions, la présence d'un ou plusieurs corps gras non peroxydés permet de réduire la toxicité des composés de dégradation secondaire de la peroxydation tels que les aldéhydes contenus dans les formulations décrites, en particulier le malondialdéhyde (MDA).

La demande européenne EP-A-0 481 148 décrit des compositions pharmaceutiques, et notamment des compositions pharmaceutiques du domaine de la thérapeutique tumorale, renfermant, à titre de principes actifs, une huile hyper-oxygénée et un dérivé oxygéné du rétinène, en association avec un véhicule ou un excipient inerte non irritant pharmaceutiquement compatible. Dans ces compositions, l'huile hyper-oxygénée est utilisée à des doses toujours inférieures à 5 % en poids et leur but est de favoriser le passage du dérivé du rétinène dans le derme et sa fixation sur les lésions à traiter.

Il a maintenant été découvert de façon tout à fait surprenante qu'il était possible de diminuer considérablement le caractère irritant d'un principe actif, notamment d'un principe actif pharmaceutique, en particulier d'un principe actif dermatologique, à application topique en appliquant simultanément sur la peau une quantité suffisamment importante d'une composition contenant une quantité suffisamment importante de lipides peroxydés.

Ainsi, la présente invention concerne une nouvelle utilisation selon la revendication 13 des lipides peroxydés, en particulier des huiles peroxydées, en tant qu'agent destiné à prévenir et/ou traiter les effets irritants d'un principe actif.

Selon un aspect particulièrement intéressant, l'invention concerne des compositions, notamment des compositions pharmaceutiques, renfermant à la fois le principe actif irritant et une quantité suffisante de lipides peroxydés.

Ainsi donc, selon un premier aspect, l'invention concerne de nouvelles compositions selon la revendication 1 pour application topique sur la peau, contenant un principe actif, notamment un principe actif pharmaceutique, à caractère irritant dans un véhicule cosmétiquement ou pharmaceutiquement acceptable contenant plus de 5 % en poids de lipides peroxydés.

Etant donné le caractère sévère des phénomènes d'irritation que se propose de résoudre la présente invention, les compositions visées par la présente invention sont généralement des compositions du domaine pharmaceutique, en particulier du domaine dermatologique.

Les lipides peroxydés dont l'utilisation est revendiquée selon la présente invention résultent de la peroxydation de corps gras insaturés.

Le degré de peroxydation est mesuré selon la norme ISO 3960.

Pour la mise en oeuvre de la présente invention, on choisira avantageusement des lipides peroxydés présentant un taux de peroxydation compris entre 5 et 600 milli-équivalents par kilo, de préférence entre 30 et 500 milli-équivalents par kilo.

Ce taux de peroxydation sera de façon encore plus avantageuse compris entre 50 et 300 milli-équivalents par kilo, de préférence encore entre 50 et 150 milli-équivalents par kilo.

Les lipides peroxydés préférés utilisés selon l'invention résultent de la peroxydation de lipides d'origine végétale, de préférence de lipides issus d'une huile végétale naturelle.

A titre d'exemples d'huile naturelle choisie selon l'invention, on citera, l'huile d'amande douce, l'huile de noisette, l'huile d'arachide, l'huile de maïs, l'huile de pépin de raisin, l'huile de sésame et l'huile de carthame. On pourra également utiliser un mélange de ces huiles.

Selon une variante particulièrement préférée de l'invention, on choisira l'huile de maïs peroxydée et tout particulièrement une huile de maïs présentant un taux de peroxydation compris entre 500 et 600 mmili-équivalents par kilo.

Les lipides peroxydés utilisés selon l'invention sont d'une façon avantageuse constitués, à titre de constituants majoritaires, représentant généralement au moins 80 % de la masse de triglycérides répondant à la formule 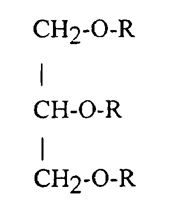 dans laquelle les radicaux R sont majoritairement représentés par des acides insaturés en C₁₈ partiellement peroxydés (en fonction du taux de peroxydation dudit lipide).

Les compositions de l'invention peuvent être sous toute forme compatible avec une application topique à la surface de la peau. Ces compositions se trouveront de préférence sous la forme d'une émulsion de type huile-dans-eau ou eau-dans-huile, d'une crème, d'une pommade, d'un lait ou d'un gel, en particulier d'un gel huileux à base de silice colloïdale.

Les compositions de l'invention contiennent, pour que l'effet du lipide peroxydé soit sensible et permette de prévenir ou traiter efficacement l'effet irritant du principe actif, plus de 5 % en poids de ce lipide peroxydé.

Selon une variante avantageuse de l'invention, la concentration en lipides peroxydés dans la composition est comprise entre 5 (valeur exclue) et 97 % en poids, de préférence entre 10 et 95 %.

La concentration des compositions de l'invention en lipides peroxydés dépend à la fois de la nature du principe actif à caractère irritant et de son degré d'agressivité vis-à-vis de la peau. Elle dépend donc à la fois de la concentration et de la nature du principe actif. Toutefois, il est clair que, pour un même principe actif, cette concentration sera d'autant plus élevée que la concentration du principe actif dans la composition sera elle-même plus élevée.

Par ailleurs, et cela constitue un avantage supplémentaire de la composition selon l'invention, la présence dans ladite composition de lipides peroxydés en quantité suffisante permet, si nécessaire, d'augmenter sensiblement les concentrations habituelles en principe actif, et cela, sans avoir de risques supplémentaires d'irritation.

Comme exposé précédemment, l'invention s'adresse notamment aux principes actifs irritants des compositions connues dans le domaine pharmaceutique, notamment dans le domaine dermatologique.

A titre de principe actif particulièrement irritant, pour lesquels la présente invention apporte une solution tout particulièrement efficace, on citera la capsicine, les rétinoïdes de synthèse, l'acide rétinoïque, le peroxyde de benzoyle, la nicotinamide, l'acide salicylique, l'hydroquinone et le 5-fluoro uracyl.

Comme cela ressort en particulier des exemples qui suivent, l'invention s'est avérée tout particulièrement efficace dans le cas de la capsicine.

Selon une variante particulièrement intéressante de l'invention, dans le cas de la capsicine, la composition se trouve sous forme d'une émulsion de type eau-dans-huile ou huile-dans-eau contenant plus de 5 et au plus 50 % en poids d'huile peroxydée et de 0,010 % à 0,080 % de capsicine pure.

Selon une autre variante de l'invention et, toujours dans le cas de la capsicine, les compositions de l'invention pourront se trouver sous la forme d'un gel huileux, notamment d'un gel à base de silice colloïdale et contiennent dans ce cas avantageusement de 40 à 97 % en poids d'huile peroxydée et de 0,010 % à 0,08 % de capsicine pure.

Un autre principe actif pour lequel l'invention apporte une solution particulièrement intéressante pour éviter son caractère irritant de la peau est l'acide rétinoïque.

Dans le cas de l'acide rétinoïque, la composition sera avantageusement sous forme d'une émulsion contenant, en poids, de préférence de 0,010 % à 0,07 % d'acide rétinoïque pur. La concentration en huile peroxydée de ces compositions est supérieure à 5 % en poids et, de préférence, comprise entre 7 % et 90 %, de préférence entre 10 et 50 %.

Toujours dans le cas de l'acide rétinoïque, les compositions pourront également se trouver sous forme de gel, en particulier de gels contenant de 0,010% à 0,07% en poids d'acide rétinoïque pur pour 40 à 97% d'huile peroxydée.

Bien entendu, les compositions de l'invention pourront en outre comprendre différents additifs classiquement utilisés dans ce type de compositions à usage topique, en particulier en dermatologie.

Ainsi donc, les compositions de l'invention pourront contenir en outre des agents émulsionnants, des parfums, des conservateurs du type de ceux classiquement utilisés dans les compositions à usage topique en dermatologie.

Comme exposé précédemment, l'invention fournit un moyen particulièrement efficace de prévenir ou traiter tous les phénomènes d'intolérance liés à l'usage de produits pharmaceutiques, notamment dermatologiques, connus pour leurs caractéristiques irritantes ou très irritantes, les huiles peroxydées agissant par une action anti-érythémateuse ou anti-réactionnelle, permettant notamment d'éviter toute réaction eczémateuse ainsi que tout risque de formation de cloques ou de vésicules à la surface de la peau lors de l'application d'un principe actif connu pour son caractère particulièrement irritant.

L'invention offre donc un moyen de lutter efficacement contre de telles réactions en appliquant sur la peau, simultanément l'huile peroxydée en quantité suffisante et le principe actif, en vue d'éviter les désagréments liés à l'agressivité de ce principe actif.

L'invention couvre donc tous les procédés de traitement topique de la peau dans lesquels on applique la composition de la revendication 1 contenant principe actif dont le caractère irritant est connu et une quantité suffisante d'huile peroxydée pour éviter tous les désagréments liés à l'application de ce principe actif sur la peau.

Ainsi donc, l'invention vise également, selon un deuxième aspect les utilisations selon la revendication 13 d'un lipide peroxydé tel qu'il a été défini précédemment pour la préparation d'une composition pharmaceutique à usage topique destinée à prévenir et/ou à traiter des irritations de la peau dues à un principe actif pharmaceutique, notamment dermatologique, inclus dans ladite composition.

Cette utilisation permet notamment de prévenir et/ou d'éviter les manifestations eczémateuses, les formations de cloques ou de vésicules ainsi que toute action érythémateuse liée à l'application topique sur la peau dudit principe actif.

Comme exposé précédemment, cet effet pourra être obtenu en appliquant sur la peau la composition de la revendication 1 renfermant l'huile peroxydée et le principe actif dont on veut conjurer l'effet irritant.

Les compositions utilisables pour la mise en oeuvre de ce procédé de traitement, contenant le principe actif dont on veut éviter les effets secondaires peuvent être sous l'une des formes définies précédemment, en particulier sous forme de gel ou d'émulsion et contenir différents additifs classiquement utilisés dans le domaine de la dermatologie.

Les exemples qui suivent sont donnés à titre purement illustratif de la présente invention.

### EXEMPLES

### Exemple 1

### Mise en évidence de l'effet des huiles peroxydées sur la tolérance de la capsicine

L'objet de l'étude réalisée selon cet exemple est de comparer la tolérance cutanée d'un produit selon l'invention avec celle d'un produit en tout point identique mais dans lequel l'huile peroxydée a été remplacée par de l'huile de vaseline.

Cette étude comparative a été réalisée en procédant selon un premier test dit test "pilote" et un deuxième test dit "test dermatologique" sur une durée plus longue de 4 jours, effectué sous contrôle dermatologique.

Les protocoles des deux tests sont donnés ci-dessous.

### 1) Protocole des tests utilisés

### a) Protocole de l'étude « pilote »

### - Critère d'inclusion :

Hommes ou femmes adultes en bonne santé

### - Critère d'exclusion :

Toute dermatose
Toute intolérance aux huiles peroxygénées

### - mode d'application :

L'expérimentateur applique lui-même une noisette du produit 1 sur la moitié inférieure latérale gauche du cou du volontaire jusqu'à la jonction avec l'épaule. L'expérimentateur masse fermement de l'oreille jusqu'à l'épaule pendant au moins 10 à 25 s jusqu'à absorption complète du produit 1 par la peau.

L'expérimentateur se lave ensuite les mains puis il applique une noisette du produit 2 sur la moitié inférieure latérale droite du cou du volontaire jusqu'à la jonction avec l'épaule. L'expérimentateur masse ensuite fermement de l'oreille jusqu'à l'épaule pendant au moins 20 à 25 s jusqu'à l'absorption complète du produit 2 par la peau.

Après 5 min, le volontaire remplit une première partie de la fiche d'observation qui lui est remise en précisant le niveau de l'effet piquant éventuel du produit ressenti ainsi que le niveau de la sensation de chaleur éventuellement ressentie. Pour chacun de ces effets, le volontaire a le choix entre 4 niveaux : aucun, très léger, évident, intense.

L'expérimentateur, quant à lui, remplit la deuxième partie de la fiche d'observation relative à l'apparition éventuelle et à l'intensité d'un érythème en classant de la même façon le niveau (aucun, très léger, évident, intense).

### b) Protocole du test "dermatologique"

Un deuxième test dit test "dermatologique" a été réalisé sur la même base mais avec une durée plus longue de 4 jours (de J1 à J4) avec 3 applications par jour.
Plus précisément, dans ce test, les produits sont appliqués trois fois par jour sur le côté du cou concerné de la manière suivante : application d'une noisette de produit sur la moitié inférieure latérale du cou, jusqu'à la jonction avec l'épaule et massage pendant 20 à 25 secondes jusqu'à pénétration du produit sur la partie du cou latérale, en partant de l'oreille jusqu'à la jonction du cou à l'épaule.

Aucun autre produit ne doit être appliqué au niveau du cou excepté les produits de toilette

### A J1:

- Les volontaires viennent au laboratoire, ils sont informés du déroulement de l'étude et remplissent une fiche de consentement et une fiche d'information en double exemplaire.
- Un examen clinique est réalisé au niveau des deux cotés du cou sous contrôle dermatologique.
- Application des deux produits (un sur chaque face latérale du cou) par la Technicienne.
- 5 minutes après la fin de l'application, la Technicienne interroge les volontaires sur les sensations qu'ils perçoivent.
   Ces sensations sont évaluées selon l'échelle suivante :
   0 : nulle
   1 : très légère
   2 : légère
   3 : modérée
   4 : intense
   5 : très intense
- Distribution des produits que les volontaires appliquent chez eux selon le même protocole.
- Les volontaires font deux autres applications dans la journée et remplissent deux fiches d'évaluation 5 minutes après application (en se lavant les mains entre chaque application sur chaque hémi-cou).

### A J2 et à J3 :

- Trois applications dans la journée de chaque produit selon le même protocole.
- 5 minutes après la fin de chaque application, les volontaires remplissent les fiches d'évaluation.

### A J4 :

- Les volontaires viennent au laboratoire et rapportent les produits et les fiches d'évaluation.
- Dernière application par la Technicienne.
- 5 minutes après la fin de l'application, la Technicienne les interroge sur les sensations qu'ils perçoivent.

### 2) Produits testés

Les produits testés dans cet exemple selon les deux protocoles ci-dessus ont la composition donnée dans les deux tableaux ci-dessous :
- Produit 1 : gel à 0,075 % en poids de capsicine pure en présence d'huile vaseline fluide (comparatif),
- Produit 2 : gel à 0,075 % en poids de capsicine pure en présence d'huile de maïs peroxydée désodorisée (exemple selon l'invention).

**TABLEAU 1 (Produit 1)**

| MATIERES PREMIERES | % en poids | Quantités pesées pour 3000 g (g) |
|---|---|---|
| Vaseline | 91,925 | 2535 |
| Aérosil^{®} 300 | 7 | 210 |
| Pronalen capsicum à 1% de capsicine | | 225 |
| soit en capsicine pure sur le produit fini | 0,075 | |
| Parfum | 1 | 30 |

**TABLEAU 2 (Produit 2)**

| MATIERES PREMIERES | % en poids | Quantités pesées pour 3000 g (g) |
|---|---|---|
| Huile de maïs peroxydée désodorisée | 90,925 | 2505 |
| Aérosil^{®} 300 | 7 | 210 |
| Pronalen capsicum à 1% de capsicine | | 225 |
| soit en capsicine pure sur le produit fini | 0,075 | |
| Parfum | 2 | 60 |

### 3) Résultats

### a) Test pilote

Le test «pilote» a été réalisé sur 16 personnes selon le protocole donné ci-dessus.

Les tableaus 3, 4 et 5 ci-dessous donnent respectivement les niveaux obtenus en ce qui concerne l'effet piquant, la sensation de chaleur et le niveau d'érythème pour le produit selon l'invention (produit 2) en comparaison avec le produit 1 contenant de la vaseline en remplacement de l'huile peroxydée.

**TABLEAU 3 : effet piquant**

| | Aucun | Très léger | Evident | Intense | TOTAL |
|---|---|---|---|---|---|
| Produit 1 | 0 | 1 | 12 | 3 | 16 |
| Produit 2 | 14 | 2 | 0 | 0 | 16 |

**TABLEAU 4 : Sensation de chaleur**

| | Aucune | Très légere | Evidente | Intense | TOTAL |
|---|---|---|---|---|---|
| Produit 1 | 7 | 9 | 0 | 0 | 16 |
| Produit 2 | 15 | 1 | 0 | 0 | 16 |

**TABLEAU 5 : érythème**

| | Aucun | Très léger | Evident | Intense | TOTAL |
|---|---|---|---|---|---|
| Produit 1 | 4 | 3 | 7 | 2 | 16 |
| Produit 2 | 15 | 1 | 0 | 0 | 16 |

Il apparaît clairement que les huiles peroxydées permettent de diminuer beaucoup plus nettement les effets secondaires de la capsicine, que la vaseline.

### b) Le test « dermatologique » a été appliqué à 40 volontaires sains, un de sexe masculin et 39 de sexe féminin, d'âge compris entre 18 et 57 ans (âge moyen 33 ± 2 ans) qui ont participé à cette étude.

Ce test a été suivi d'une exploitation statistique des résultats et a permis de confirmer en tout point les résultats observés dans le test « pilote ». Plus précisément :
à chaque application, les picotements ressentis avec le produit 1 sont nettement supérieurs à ceux ressentis avec le produit 2,
de même les érythèmes constatés sont nettement plus importants avec le produit 1 qu'avec le produit 2.

La sensation de chaleur est également nettement supérieure avec le produit 1 et les démangeaisons ont été le plus souvent supérieures avec le produit 1.

Ainsi donc, pour tous les paramètres étudiés, le produit 1 a procuré des sensations nettement plus intenses que le produit 2. Ce deuxième test a donc permis de confirmer les résultats du test pilote.

### Exemple 2

### Mise en évidence de l'effet de la présence d'une huile peroxydée sur la tolérance de l'acide rétinoïque

### a) Protocole du test

La méthode utilisée selon ce test est celle décrite par PHILLIPS et al dans Toxic and Applied Pharmacology, 21:369-382, 1972.

Selon cette méthode, les produits à tester sont incorporés dans des patches. Les patches sont renouvelés quotidiennement, 5 jours par semaine, pendant 21 jours au même endroit de la peau.

Après avoir enlevé le patch, on effectue une lecture du caractère irritant du produit en attribuant une note comprise entre 0 et 4 selon l'échelle ci-dessous :
0 = aucun effet
0,5 = réaction douteuse
1 = érythème
2 = érythème et induration
3 = érythème et induration et vésicules
4 = cloques

On calcule pour chaque produit testé une note cumulative qui est la somme des notes obtenues pendant les 21 jours du test.

### b) Produits testés :

4 produits ont été comparés dans le cadre de l'étude et appliqués chacun dans les mêmes conditions aux individus ayant participé à cette étude. Il s'agit de :
- Produit n° 1 : un gel contenant, en poids, 90 % d'huile peroxydée et 0,05 % d'acide rétinoïque
- Produit n° 2 : le produit commercialisé par les Laboratoires ORTHO sous la marque RETIN-A-® Micro à 0,1 % d'acide rétinoïque
- Produit n° 3 : le produit commercialisé par les Laboratoires ORTHO sous la marque RETIN-A-® Gel à 0,025 % d'acide rétinoïque
- Produit n° 4 : le produit commercialisé par les Laboratoires ORTHO sous la marque RETIN-A-® Gel à 0,01 % d'acide rétinoïque

### c) Résultats obtenus

Le test a été effectué, selon le protocole ci-dessus, sur 25 sujets de plus de 18 ans en bonne santé.

Les résultats cumulatifs (somme des notes attribuées quotidiennement, pour chaque produit, sur les 25 sujets ayant participé à l'étude), observés au bout de 21 jours selon l'échelle de lecture donnée précédemment, ont été les suivants :
- Produit 1 : 0
- Produit 2 : 15
- Produit 3 : 80
- Produit 4 : 54

Cette étude montre donc clairement que, dans les conditions du test, le produit selon l'invention s'est avéré beaucoup moins irritant que les produits comparatifs.

### Exemple 3

### Bases de formulation utilisable comme véhicule pour les compositions de l'invention

Les proportions dans les exemples qui suivent sont données en pourcentage en poids.

| a) Préparation liquide huileuse | |
|---|---|
| | |
| Huile végétale peroxydée | 45 % |
| Huile de vaseline fluide | 34,1 % |
| Parahydroxybenzoate de propyle | 0,2 % |
| Stéarine | 8 % |
| Huile silicone | 10,7 % |
| Parfum | 2 % |
| | |

| b) Lotion huileuse | |
|---|---|
| | |
| Huile végétale peroxydée | 36 % |
| Huile de noisette | 60 % |
| Parfum | 4 % |
| | |

| c) Lotion hydroalcoolique | |
|---|---|
| | |
| Huile végétale peroxydée | 10 % |
| Dragoxat-2 éthyléthyl-2-éthylhexanate | 3 % |
| Butyl hydroxyanisol | 0,01 % |
| Alcool dénaturée 99.9 | 23 % |
| Parfum | 0,10 % |
| Eau déminéralisée | QSP 100 |
| | |

| d) Emulsion eau-dans-huile | |
|---|---|
| | |
| Huile végétale peroxydée | 30 % |
| Glycérides semi synthétiques | 30 % |
| Eau | QSP 100 |

| e) Crème contenant une émulsion de type huile-dans-eau | |
|---|---|
| | |
| Huile végétale peroxydée | 20 % |
| Copolymères acryliques anioniques en | |
| dispersion dans huile blanche | 4,00 % |
| Parahydroxybenzoate de méthyl sodé | 0,15 % |
| Parahydroxybenzoate de propyl sodé | 0,05 % |
| Méthylchloroisothiazoline- | 0,0012 % |
| méthylisothiazolinone | |
| Parfum | 0,05 % |
| Eau déminéralisée | QSP 100 |
| | |

| f) Gel aqueux | |
|---|---|
| | |
| Uvinul MS 40 (Laserson) | 0,050 |
| Triéthanolamine 99 | 0,5 % |
| Carbopol 934 solution à 2 % | 25 % |
| Huile végétale peroxydée | 10 % |
| Alcool éthylique dénaturé 95/96 % | 40 % |
| Parfum | 0,30 % |
| Eau déminéralisée | Q.S.P. 100 |
| | |

| g) Crème contenant une émulsion de type huile-dans-eau | |
|---|---|
| | |
| Huile végétale peroxydée | 25 % |
| Salcare SC 81 | 6 % |
| Parfum | 1 % |
| Triéthanolamine | 0,85 % |
| Para hydroxybenzoate de méthyle | 0,15 % |
| Para hydroxybenzoate de propyle | 0,05 % |
| Chlorométhylchloroisothiazoline, | |
| Chlorométhylisothiazolinone. | 0,05 % |
| Eau déminéralisée | Q.S.P.100 |

| h) Gel huileux | |
|---|---|
| | |
| Huile végétale peroxydée | 92,20 % |
| Aerosil 300 | 7 % |
| Arôme ; Parfum | 0,8 % |

## Revendications

1. Composition pour application topique sur la peau, contenant un principe actif, notamment un principe actif pharmaceutique, à caractère irritant dans un véhicule cosmétiquement ou pharmaceutiquement acceptable, **caractérisée en ce que** ledit véhicule contient plus de 5 % en poids de lipides peroxydés, **en ce que** ladite composition contient plus de 5 % en poids desdits lipides peroxydés et **en ce que** ledit principe actif à caractère irritant est choisi dans le groupe constitué de la capsicine, des rétinoïdes de synthèse, de l'acide rétinoïque, du peroxyde de benzoyle, de la nicotinamide, de l'acide salicylique, de l'hydroquinone, et du 5-fluoro uracyl.

2. Composition selon la revendication 1, **caractérisée en ce que** lesdits lipides peroxydés présentent un taux de peroxydation compris entre 5 et 600, de préférence entre 30 et 500, de préférence encore entre 50 et 300, de préférence encore entre 50 et 150 milli-équivalents par kilo.

3. Composition selon l'une des revendications 1 ou 2, **caractérisée en ce que** lesdits lipides peroxydés comprennent, à titre de constituants majoritaires, des triglycérides partiellement oxydés répondant à la formule générale : 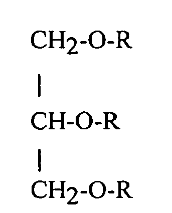 dans laquelle les radicaux R sont des acides insaturés en C₁₈ partiellement peroxydés.

4. Composition selon l'une des revendications 1 à 3, **caractérisée en ce que** lesdits lipides peroxydés sont obtenus par peroxydation de lipides d'origine végétale, de préférence de lipides issus d'une huile végétale naturelle.

5. Composition selon la revendication 4, **caractérisée en ce que** l'huile naturelle est choisie dans le groupe constitué de l'huile d'amande douce, de l'huile de noisette, de l'huile d'arachide, de l'huile de maïs, de l'huile de pépin de raisin, de l'huile de sésame et de l'huile de carthame et de leurs mélanges.

6. Composition selon 'une des revendications 1 à 5, **caractérisée en ce qu'**elle est sous la forme d'une émulsion de type huile-dans-eau ou eau-dans-huile, d'une crème, d'une pommade, d'un lait ou d'un gel, en particulier d'un gel huileux à base de silice colloïdale.

7. Composition selon l'une des revendications 1 à 6, **caractérisée en ce qu'**elle contient à titre de principe actif de la capsicine.

8. Composition selon la revendication 7, **caractérisée en ce qu'**elle est sous la forme d'une émulsion de type eau-dans-huile ou huile-dans-eau contenant plus de 5 % et au plus 50 % en poids d'huile peroxydée et de 0,010 % à 0,080 % de capsicine pure.

9. Composition selon la revendication 7, **caractérisée en ce qu'**elle est sous la forme d'un gel huileux, notamment d'un gel à base de silice colloïdale, et contient de 40 à 97 % en poids d'huile peroxydée et de 0,010 % à 0,08 % de capsicine pure.

10. Composition selon l'une des revendications 1 à 6, **caractérisée en ce que** le principe actif est de l'acide rétinoïque.

11. Composition selon la revendication 10, **caractérisée en ce qu'**elle est sous la forme d'une émulsion contenant en poids, de préférence 0,010 % à 0,07 % d'acide rétinoïque pur, et plus de 5 %, de préférence 7 à 90 %, d'huile peroxydée.

12. Composition selon la revendication 10, **caractérisée en ce qu'**elle est sous la forme d'un gel contenant de 0,010 % à 0,07 % en poids d'acide rétinoïque pur pour 40 à 97 % d'huile peroxydée.

13. Utilisation d'un lipide peroxydé tel que défini dans l'une des revendications 1 à 5, pour la préparation d'une composition pharmaceutique à usage topique contenant plus de 5 % en poids dudit lipide peroxydé, destinée à prévenir et/ou à traiter l'irritation de la peau due à un principe actif pharmaceutique, notamment dermatologique inclus dans ladite composition et choisi dans le groupe constitué de la capsicine, des rétinoïdes de synthèse, de l'acide rétinoïque, du peroxyde de benzoyle, de la nicotinamide, de l'acide salicylique, de l'hydroquinone, et du 5-fluoro uracyl.

14. Utilisation selon la revendication 13, **caractérisée en ce que** ladite composition est destinée à prévenir et/ou à éviter les manifestations eczémateuses, les formations de cloques ou de vésicules ainsi que toute action érythémateuse liée à l'application topique sur la peau dudit principe actif irritant.

15. L'utilisation selon l'une des revendications 13 ou 14, **caractérisée en ce que** ledit lipide peroxydé est tel que défini dans l'une des revendications 2 à 5.

16. Utilisation selon l'une des revendications 13 à 15, **caractérisée en ce que** ledit principe actif irritant est inclus dans ladite composition pharmaceutique, cette dernière étant telle que définie selon l'une des revendications 1 à 12.

## Claims

1. Composition for topical application to the skin, containing an active principle, especially an active pharmaceutical principle, with irritant character in a cosmetically or pharmaceutically acceptable vehicle, **characterized in that** said vehicle contains more than 5% by weight of peroxidized lipids, **in that** said composition contains more than 5% by weight of said peroxidized lipids, and **in that** said active principle with irritant character is chosen within the group constituted of capsaicin, synthetic retinoids, retinoic acid, benzoyl peroxide, nicotinamide, salicylic acid, hydroquinone and 5-fluorouracil.

2. Composition according to Claim 1, **characterized in that** said peroxidized lipids have a peroxidation level of between 5 and 600, preferably between 30 and 500, more preferably between 50 and 300, more preferably between 50 and 150 milliequivalents per kilo.

3. Composition according to either of Claim 1 or 2, **characterized in that** said peroxidized lipids comprise, as major constituent, partially oxidized triglycerides corresponding to the general formula: 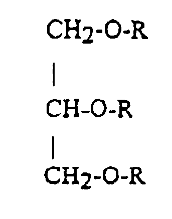 in which the radicals R are partially peroxidized C₁₈ unsaturated acids.

4. Composition according to one of Claims 1 to 3, **characterized in that** said peroxidized lipids are obtained by peroxidation of lipids of vegetable origin, preferably of lipids from a natural vegetable oil.

5. Composition according to Claim 4, **characterized in that** the natural oil is chosen within the group constituted of sweet almond oil, hazelnut oil, peanut oil, corn oil, grapeseed oil, sesame oil and safflower oil and mixtures thereof.

6. Composition according to one of Claims 1 to 5, **characterized in that** it is in the form of an emulsion of oil-in-water or water-in-oil type, a cream, a lotion, a milk or a gel, in particular an oily gel based on colloidal silica.

7. Composition according to one of Claims 1 to 6, **characterized in that** it contains capsaicin as active principle.

8. Composition according to Claim 7, **characterized in that** it is in the form of an emulsion of water-in-oil or oil-in-water type containing more than 5% and at most 50% by weight of peroxidized oil and from 0.010% to 0.080% of pure capsaicin.

9. Composition according to Claim 7, **characterized in that** it is in the form of an oily gel, especially of a gel based on colloidal silica, and contains from 40 to 97% by weight of peroxidized oil and from 0.010% to 0.08% of pure capsaicin.

10. Composition according to one of Claims 1 to 6, **characterized in that** the active principle is retinoic acid.

11. Composition according to Claim 10, **characterized in that** it is in the form of an emulsion containing preferably 0.010% to 0.07% by weight of pure retinoic acid, and more than 5%, preferably 7 to 90% by weight, of peroxidized oil.

12. Composition according to Claim 10, **characterized in that** it is in the form of a gel containing from 0.010% to 0.07% by weight of pure retinoic acid for 40 to 97% of peroxidized oil.

13. Use of a peroxidized lipid such as defined in one of Claims 1 to 5, for the preparation of a pharmaceutical composition for topical use containing more than 5% by weight of said peroxidized lipid, intended to prevent and/or to treat irritation of the skin due to a pharmaceutical, especially dermatological, active principle included in said composition and chosen within the group formed of capsaicin, synthetic retinoids, retinoic acid, benzoyl peroxide, nicotinamide, salicylic acid, hydroquinone and 5-fluorouracil.

14. Use according to Claim 13, **characterized in that** said composition is intended to prevent and/or to avoid eczematous manifestations, the formation of blisters or of vesicles as well as any erythematous action connected with the topical application on the skin of said irritant active principle.

15. Use according to either of Claims 13 and 14, **characterized in that** said peroxidized lipid is as defined in one of Claims 2 to 5.

16. Use according to one of Claims 13 to 15, **characterized in that** said irritant active principle is included in said pharmaceutical composition, the latter being as defined in one of Claims 1 to 12.

## Patentansprüche

1. Zusammensetzung zur topischen Anwendung auf der Haut, enthaltend einen Wirkstoff, vor allem einen pharmazeutischen Wirkstoff, mit reizender Eigenschaft in einem kosmetisch oder pharmazeutisch akzeptablen Vehikel, **dadurch gekennzeichnet, daß** das Vehikel mehr als 5 Gew.-% peroxidierte Lipide enthält, daß die Zusammensetzung mehr als 5 Gew.-% der peroxidierten Lipide enthält und daß der Wirkstoff mit reizender Eigenschaft aus der Gruppe bestehend aus Capsicin, Synthese-Retinoiden, Retinsäure, Benzoylperoxid, Nicotinamid, Salicylsäure, Hydrochinon und 5-Fluorouracil ausgewählt ist.

2. Zusammensetzung nach Anspruch 1, **dadurch gekennzeichnet, daß** die peroxidierten Lipide eine Peroxidzahl zwischen 5 und 600, vorzugsweise zwischen 30 und 500, weiterhin vorzugsweise zwischen 50 und 300, weiterhin vorzugsweise zwischen 50 und 150 Milliäquivalente pro Kilogramm aufweisen.

3. Zusammensetzung nach einem der Ansprüche 1 oder 2, **dadurch gekennzeichnet, daß** die peroxidierten Lipide als Hauptbestandteile partiell oxidierte Triglyceride umfassen, die der folgenden allgemeinen Formel entsprechen: 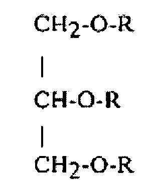 worin die Radikale R teilweise peroxidierte ungesättigte C₁₈-Säuren sind.

4. Zusammensetzung nach einem der Ansprüche 1 bis 3, **dadurch gekennzeichnet, daß** die peroxidierten Lipide durch Peroxidation von Lipiden pflanzlichen Ursprungs, vorzugsweise von Lipiden aus einem natürlichen Pflanzenöl erhalten werden.

5. Zusammensetzung nach Anspruch 4, **dadurch gekennzeichnet, daß** das natürliche Öl aus der Gruppe bestehend aus Süßmandelöl, Haselnußöl, Erdnußöl, Maisöl, Traubenkernöl, Sesamöl und Distelöl sowie deren Mischungen ausgewählt ist.

6. Zusammensetzung nach einem der Ansprüche 1 bis 5, **dadurch gekennzeichnet, daß** sie in Form einer Emulsion von der Art Öl-in-Wasser- oder Wasser-in-Öl-Emulsion, einer Creme, einer Salbe, einer Milch oder eines Gels, insbesondere eines öligen Gels auf der Basis von kolloidalem Siliziumdioxid vorliegt.

7. Zusammensetzung nach einem der Ansprüche 1 bis 6, **dadurch gekennzeichnet, daß** sie als Wirkstoff Capsicin enthält.

8. Zusammensetzung nach Anspruch 7, **dadurch gekennzeichnet, daß** sie in Form einer Emulsion von der Art Wasser-in-Öl- oder Öl-in-Wasser-Emulsion vorliegt, die mehr als 5 und höchstens 50 Gew.-% peroxidiertes Öl sowie zwischen 0,010 % und 0,080 % reines Capsicin enthält.

9. Zusammensetzung nach Anspruch 7, **dadurch gekennzeichnet, daß** sie in Form eines öligen Gels, insbesondere eines Gels auf der Basis von kolloidalem Siliziumdioxid vorliegt und zwischen 40 und 97 Gew.-% peroxidiertes Öl sowie zwischen 0,010 % und 0,08 % reines Capsicin enthält.

10. Zusammensetzung nach einem der Ansprüche 1 bis 6, **dadurch gekennzeichnet, daß** der Wirkstoff Retinsäure ist.

11. Zusammensetzung nach Anspruch 10, **dadurch gekennzeichnet, daß** sie in Form einer Emulsion vorliegt, die vorzugsweise 0,010 bis 0,07 Gew.-% reine Retinsäure und mehr als 5, vorzugsweise 7 bis 90 Gew.-% peroxidiertes Öl enthält.

12. Zusammensetzung nach Anspruch 10, **dadurch gekennzeichnet, daß** sie in Form eines Gels vorliegt, das zwischen 0,010 und 0,07 Gew.-% reine Retinsäure bei 40 bis 97 % peroxidiertem Öl enthält.

13. Verwendung eines peroxidierten Lipids wie es in einem der Ansprüche 1 bis 5 definiert ist, für die Zubereitung einer pharmazeutischen Zusammensetzung mit topischer Anwendung, die mehr als 5 Gew.-% des peroxidierten Lipids enthält und zur Vorbeugung und/oder zur Behandlung der Hautirritation bestimmt ist, die durch einen pharmazeutischen, insbesondere dermatologischen Wirkstoff bedingt ist, welcher in der Zusammensetzung enthalten und aus der Gruppe bestehend aus Capsicin, Synthese-Retinoiden, Retinsäure, Benzoylperoxid, Nicotinamid, Salicylsäure, Hydrochinon und 5-Fluorouracil ausgewählt ist.

14. Verwendung nach Anspruch 13, **dadurch gekennzeichnet, daß** die Zusammensetzung zur Vorbeugung und/oder zur Vermeidung von ekzematösen Erscheinungen, Blasen- oder Bläschenbildungen sowie jedweder erythematösen Wirkung, die mit der topischen Anwendung des reizenden Wirkstoffs auf der Haut in Verbindung steht, bestimmt ist.

15. Verwendung nach einem der Ansprüche 13 oder 14, **dadurch gekennzeichnet, daß** das peroxidierte Lipid derart ist, wie es in einem der Ansprüche 2 bis 5 definiert ist.

16. Verwendung nach einem der Ansprüche 13 bis 15, **dadurch gekennzeichnet, daß** der reizende Wirkstoff in der pharmazeutischen Zusammensetzung enthalten ist, wobei letztere derart ist, wie sie nach einem der Ansprüche 1 bis 12 definiert ist.
